# EUROPEAN PATENT SPECIFICATION

(11) **EP 3 595 632 B1**
(45) Date of publication and mention of the grant of the patent: **28.04.2021**
(21) Application number: 18715807.6
(22) Date of filing: 12.03.2018
(51) Int. Cl.: A61K 31/352, A61K 36/9066, A61K 36/28, A61K 31/201, A61P 15/00, A23L 2/52, A23L 33/10, A23L 33/105, A23L 33/115, A61K 31/12, A61K 31/202, A61K 31/519, A61K 45/06

(54) **COMPOSITION FOR USE IN THE TREATMENT OF ENDOMETRIOSIS AND SYMPTOMS ASSOCIATED WITH ENDOMETRIOSIS**
ZUSAMMENSETZUNG ZUR VERWENDUNG BEI DER BEHANDLUNG VON ENDOMETRIOSE UND SYMPTOMEN IM ZUSAMMENHANG MIT ENDOMETRIOSE
COMPOSITION DESTINÉE À ÊTRE UTILISÉE DANS LE TRAITEMENT DE L'ENDOMÉTRIOSE ET DES SYMPTÔMES ASSOCIÉS À L'ENDOMÉTRIOSE

(30) Priority: 16.03.2017 IT 201700029316
(43) Date of publication of application: 22.01.2020
(73) Proprietor: Sistemi Salute S.r.l., 80121 Napoli (IT)
(72) Inventor: SIGNORILE, Pietro Giulio, 00189 Roma (IT)
(74) Representative: Di Giovine, Paolo
(86) International application number: PCT/IB2018/051614
(87) International publication number: WO 2018/167635

(56) References cited:
- WO-A1-2014/111268
- WO-A2-2004/004638
- US-B1- 6 534 086
- HARRIS T VLASS AM: "Can Herbal Medicines Improve Cellular Immunity Patterns in Endometriosis?", MEDICINAL AND AROMATIC PLANTS, vol. 04, no. 02, 1 January 2014 (2014-01-01), XP055415962, DOI: 10.4172/2167-0412.1000184

## Description

### FIELD OF THE INVENTION

The present invention relates to a composition for oral use, in particular in form of food supplement comprising quercetin, extract of curcuma or curcumin, extract of feverfew and a preparation comprising omega-3 and/or omega-6 and optionally nicotinamide and/or 5-methyltetrahydrofolate calcium salt. Moreover, the present invention relates to the use of such composition for the treatment of endometriosis symptoms and in reducing endometriosis outbreaks.

### STATE OF ART

Endometriosis, a disease affecting more than 150,000,000 women in Western world is a disease with chronic development therefor currently there are no medical therapies apt to reduce or eliminate the disease.

Endometriosis is characterized by disabling symptoms affecting young women with serious disabling physical discomfort, difficulties in the social life and infertility. Such symptoms are due to the inflammatory process determined by the development of the disease outbreaks most frequently at the expense of pelvic organs, genital organs, intestine and bladder, apart from the sustaining structures thereof.

The factors that are altered in acute and chronic inflammatory processes are several, a reduction in such factors reduces the inflammatory process that in endometriosis is responsible for the symptoms (acute and chronic pelvic pain, dyspareunia, dysmenorrhea, pain related to ovulation, pain for urination, pain in the bowel functions, chronic physical fatigue, headache, female infertility).

It is known that estrogenic reduction favours less disease activity with a lower host immune response which translates into less acute and chronic inflammation. The factors involved in this inflammatory process are prostaglandins PGE 2, TNF alfa, metalloproteinase, VEGF.

The estrogens, by increasing the *in vivo* activity of the disease outbreaks, increase the inflammatory response in the involved organs.

Harris and Vlass 2015 (Med Aromat Plants 2015, 4:2 "Can Herbal Medicines Improve Cellular Immunity Patterns in Endometriosis?) underline the limited information provided on the benefits of herbal medicine in the treatment of endometriosis and reviews the state of knowledge for the pathogenesis of endometriosis and the role of the immune system in the causation of the disease indicating that there are a range of herbs having the capacity to improve immunological function in endometriosis. The object of the present invention is to provide a composition alternative to the ones known in the state of art useful in the treatment of endometriosis.

### SUMMARY OF THE INVENTION

The present invention is based upon searching and identifying a new combination of natural active principles exerting effects in reducing the inflammatory response and effects in reducing the disease activity with the purpose of mitigating the inflammatory response, by reducing the symptoms produced by the disease and the dangerous effects thereof on the involved organs, both for the single actions of each single combination element and for the synergic and strengthened action of the various components of the combination the invention relates to. Moreover the combination of active ingredients of the present invention allowed to obtain a reduction in disease outbreaks, demonstrated by a decrease in the values of specific marker Ca 125. The advantageous effect is obtained at relatively low dosages of the single components, by remaining in the specification of tables of food supplements. The present invention relates to a compositions including quercetin, extract of curcuma or curcumin, extract of feverfew, and a preparation comprising omega-3 and/or omega-6 and optionally nicotinamide and/or 5-methyltetrahydrofolate calcium salt. The present invention further relates to such compositions for use in the treatment of endometriosis.

From data deriving from experiments the effectiveness and utility of such compositions is demonstrated in reducing both symptomatology by means of used parameters and the reduction in the important marker of inflammation induced by the presence of endometriosis PGE2 (prostaglandin E2). As shown by the comparison between group I treated with the combination of the above-mentioned compounds and group II treated only with a portion thereof. The treatment constituted by all combined components carried out a synergic action apt to have a statistically significant effectiveness on the symptoms of endometriosis and of its haematic parameter PGE2. Surprisingly, the combination of the composition compounds then showed a synergic effect.

Other advantages and features of the present invention will result evident from the following detailed description.

### DETAILED DESCRIPTION OF THE INVENTION

The present invention describes a composition comprising, as main active ingredients, quercetin, extract of curcuma or curcumin, extract of feverfew and a preparation comprising omega-3 and/or omega-6 and optionally nicotinamide and/or 5-methyltetrahydrofolate calcium salt.

In the present description under the term preparation including omega-3 and/or omega-6 any preparation is meant, for example of natural, animal or plant origin, including essential fatty acids omega-3 and/or omega-6, preferably both of them. According to an embodiment the composition will include a concentration of omega-3 between 200 and 3000 mg and/or omega-6 between 50 and 2000 mg, in particular per dosage unit to be administered. Examples of omega-3 and omega-6-based preparations are linseed oil, fish oil, Chia seeds, rape-seed oil. The linseed oil can be obtained by the person skilled in the art by means of conventional extraction techniques, for example it is obtained by squeezing previously dried or roasted flax seeds, preferably linseed oil in the composition will be in a concentration between 0.3 and 7 grams, in particular per dosage unit to be administered. According to an embodiment Linseed oil (Linum usitatissimum L., seeds, carrier: silicium dioxide) plv., Coating agent: Hydroxypropylmetylcellulose; Bulking agent: Dibasic calcium phosphate) will be used.

According to an embodiment the linseed oil used in the composition is titrated in linoleic acid and/or α-linolenic acid, for example α-linolenic acid between 400 mg and 2000 mg.

The composition of the invention further comprises an extract of plants belonging to the genus curcuma, in particular belonging to the species *curcuma longa.* For example the composition could include an extract of curcuma in a dose between 15 and 200 mg, preferably 20 mg, such extract will include curcuma active principle titrated for example in the above-mentioned range. Curcuma determines a reduction in the amount of estrogens, inhibition of metalloproteinases, it speeds up cellular apoptosis, it reduces TNF alfa immunity mediator, it reduces interleukin mediators, it inhibits angiogenesis by reducing VEGEF.

The composition of the invention further comprises an extract of plants belonging to the species *Tanacetum parthenium (feverfew).* According to an embodiment the composition could include an extract of feverfew titrated, for example in a dose between 3 and 100 mg, preferably 19.5 mg per dosage unit. Such extract will include parthenolide active principle titrated for example in the above-mentioned range. Feverfew inhibits prostaglandin PGE(2), and TNF alfa inhibits the proliferation of fibroblasts, it has an anti-hemicrania action and an action for reducing the inflammatory process. According to a preferred embodiment an extract of feverfew flower titrated in parthenolide between 0.3 and 0.6% will be used, such as for example the one available on the market by LCM IPA num. STP-QCPJHS404-001.

The herein described extracts could be obtained with the conventional techniques or purchase as available on the market.

The composition of the invention further comprises quercetin preferably in a dose between 50 mg and 1000 mg, in particular per dosage unit to be administered, preferably 200 mg. Quercetin reduces local estrogens by reducing FSH and LH (studies on animal model), it reduces VEGEF.

Quercetin and its obtaining modes/synthesis are widely described in the state of known art and therefore they do not require further examinations herein, by way for example it can be extracted from hawthorn, calendula, horse-chestnut tree, in particular Quercetin from flowers of *Sophora japonica* could be used.

The composition of the invention could further include nicotinamide and/or 5-methyltetrahydrofolate calcium salt. 5-methyltrahydrate calcium salt, reduces homocysteine (thromboembolic factor), whereas Nicotinamide (Vitamin B3) carries out an antiangiogenetic action. According to an embodiment the composition could include Nicotinamide, for example in a dose between 5 and 100 mg, preferably 20 mg, whereas 5-methyltetrahydrofolate calcium salt preferably in a dose between 100 mg and 600 mg.

The compositions according to the present invention can be formulated under any form and administration route and associated with any other component, in a variety of ways for example capsule, soft capsules, tablets, pills, jellies, powders or granules. Such excipients can be selected for example from those usually known in the state of art and include, but they are not limited thereto: a) carriers, such as for example sodium citrate and calcium phosphate, b) fillers such as for example starch, lactose, microcrystalline cellulose, sucrose, glucose, mannitol and colloidal silica, c) humectants, such as for example glycerol, d) disintegrant agents, such as alginate, calcium carbonate, starches, derivatives of starch, of cellulose and of polyvinylpyrrolidone, silicates and sodium carbonate e) ligands such as carboxymethylcellulose, alginates, jelly, polyvinylpyrrolidone, sucrose, polymer derivatives of cellulose, starch derivative f) retarding agents such as paraffin, cellulose polymers, esters of fatty acids g) absorption accelerators, such as quaternary ammonium compounds, h) wetting agents and surface-active agents such as cetyl alcohol and glycerol monostearate, i) adsorbents, such as benthic clays and kaolin, k) lubricants such as talcum, calcium stearate, magnesium stearate, polyethylene glycol, sodium lauryl sulphate, sodium stearyl fumarate j) glidants such as talcum, colloidal silica.

The forms of solid dosage, such as tablets, capsules, soft capsules, jellies, pills and granules, could be coated with enteric, gastric coatings or of other type known to the state of art. They could include opacifying agents and they can be of the type to allow the release of active ingredients only or preferably in a certain intestine tract, in case, in delayed way. Substances which can allow such delayed use include, but are not limited thereto, polymers and waxes.

The soft capsules could house antioxidant active substances in liquid form alone or in solutions, suspensions or emulsions of the active substances in a liquid solvent. The soft capsules could be characterized by a casing qualitatively similar to that of the stiff capsules, but thicker and softer. Liquid forms suitable to an oral for example are emulsions, solutions, prepared or extemporary suspensions, syrups and elisir. Excipients suitable to the formulations according to the present invention in liquid forms for oral use include, but they are not limited thereto, diluents such as water or other solvents, solubilising and emulsifying agents selected among ethyl alcohol, polyalcohols, propylene glycol, glycerol, polyethylenglycol and sorbitan esters. These formulations can even include sweeteners and aroma. The compositions will be for example a food supplement, a nutraceutical, dietary and nutritional composition a food product, a beverage, a neutraceutical, a medicament, a medicated food, a food for special medical purposes, a food. The compositions will be mainly intended to be used by human beings, but they could even be used on animals.

The compositions according to the present invention could include one or more suitable food ingredients and/or preservatives and/or acidifiers and/or antioxidant agents and/or immunostimulating agents and/or dyes and/or probiotic and/or prebiotic substances.

The combination of the above-mentioned active ingredients could be used formulated in one single composition according to the several above-described embodiments or in a kit including the different separated ingredients, for example in single compositions such as capsules, pills, tables for sequential or contemporary administration of the different ingredients.

The above-described compositions could be used/administered/taken for the treatment of endometriosis, in particular for the treatment of the symptoms associated to this pathology such as for example acute and chronic pelvic pain, dyspareunia, dysmenorrhea, pain related to ovulation, pain related to urination, pain in the bowel functions, chronic physical fatigue, headache, female infertility. Preferably, independently from the used formulation type, the combination of active ingredients according to the present invention will be administered with a daily dosage regime according to the above-mentioned concentrations.

### EXPERIMENTAL SECTION

### Clinical study about patients affected by endometriosis

A composition was prepared comprising linseed oil 1002 mg (alfa linolenic acid (omega 3) 432 mg and linoleic acid (omega 6) 172.8 mg), quercetin 200 mg, nicotinamide 20 mg, 5-Methyltetrahydrofolate calcium salt 400 mcg, curcuma titrated 20 mg, feverfew titrated 19.5 mg.

90 patients were identified affected by endometriosis, divided into three groups each one constituted by 30 patients. The first group of 30 patients was treated with the above-mentioned composition including all active principles. Whereas the second group of 30 patients, with a composition including only linseed oil and 5-methyltetrahydrofolate calcium salt.

The third group of 30 patients affected by endometriosis took for the same duration of the first two groups a placebo and thus had the function of control group.

The first group constituted by 30 patients took 2 capsules per day, every twelve hours, for 3 months, of the composition including all active ingredients.

The second group di 30 patients took the equivalent dose of Linseed oil 1002 mg (alfa linolenic acid (omega 3) 432 mg and linoleic acid 172.8 mg (omega 6) still twice a day for oral administration (oa) associated to 5-methyltetrahydrofolate calcium salt (400 mcg) twice a day for oral administration. These two groups of patients affected by slight or severe endometriosis diagnosticated with the diagnostic protocol constituted by a bimanual vaginal and rectal positive examination, positive Ca125 serum dosage, positive NMR (pelvic Nuclear Magnetic Resonance) and histological diagnosis at the end of intake of products performed after diagnostic and operative laparoscopic surgery, were compared to the control group constituted by 30 women affected by endometriosis, positive to the protocol like the two groups treated with the above-mentioned compositions. The groups of affected patients (60) had the disease surgical diagnosis soon after the integrator intake. Average age of affected groups I and II treated with the compositions: 34.0 years old Average age of affected control group treated with con placebo: 35.2 years old.

### Results

Symptomatology and comorbidity of the affected groups I and II group and affected placebo group each one constituted by 30 patients for a total of 90 subjects affected by endometriosis.
Cephalea: 15%
Cystitis: 12%
Muscular pain or fibromyalgia: 4%
Irritable colon: 15%
Past surgical operations: 42%
Positivity to Ca 125: 100% (exclusion criteria)
Dysmenorrhea VAS >= a 5: 62%
Dyspareunia VAS>= at 5: 37%
Chronic pelvic pain VAS>= at 5: 62%

Serum examinations performed in all three groups, group I, group II and control group:
17BEstradiol on 21^{st} gg of period before the intake in group I and II and in the last intake month 17BEstradiol on 21^{st} gg of period in the control group which was taking placebo, called Placebo group
Serum PGE2 group I, II, basal Placebo and in the last treatment month.

### Group 1

The administration of the active principles constituted by Linseed oil 1002mg (alfa linolenic acid (omega 3) 432 mg and linoleic acid 172.8 mg (omega 6)), Quercetin 200mg, Nicotinamide 20 mg, 5-Methyltetrahydrofolate calcium salt 400 mcg, Curcuma titrated 20mg, Feverfew titrated 19.5 mg, determined a reduction in symptomatology evaluated with VAS scale.

### Pre-treatment VAS scale symptomatology values

### (assimilated to placebo group)

Cephalea: 14%
Cystitis: 12%
Muscular pain or fibromyalgia: 4%
Irritable colon: 15%
Positivity to Ca 125: 100% with average value 61.4
U/ml (exclusion criterion if lower than 35 U/ml)
Dysmenorrhea VAS >= 5: 62%
Dyspareunia VAS>= 5: 30%
Chronic pelvic pain VAS>= at 5: 62%

### Serum examinations

| | |
|---|---|
| 17 Beta Estradiol (on 21^{st} day of period): | average |
| value group 1: | 184pg/ml |
| Serum dosage of PGE2 average value group 1: | 3404+/- 346 ng/l |

### 3* month treatment VAS scale symptomatology values

Cephalea: 4%
Cystitis: 2%
Muscular pain (fibromyalgia): 1%
Irritable colon: 6%
**Ca 125: 73%, average value 38 U/ml**
Dysmenorrhea VAS>= 5:**18%**
Dyspareunia VAS>= 5: 15%
Chronic pelvic pain VAS >= 5: 18%

### Serum examinations

| | |
|---|---|
| 17 Beta Estradiol (on 21^{st} day of period): | average |
| value group 1: | 171pg/ml |
| Serum dosage of PGE2 group 1: | 1377+/-326ng/l |

### Group 2

The administration of the active principles constituted by Linseed oil 1002mg (alfa linolenic acid (omega 3) 432 mg and linoleic acid 172.8 mg (omega 6)), and 5-Methyltetrahydrofolate calcium salt 400mcg, determined a reduction in the symptomatology evaluated with VAS scale and in the serum values of the marker of PGE2 inflammation, lower reduction than Group I taking all components, such differences are statistically significant and show the powerful and sole synergic action of the so-combined composition.

### Pre-treatment VAS scale values (assimilable to placebo group)

Cephalea: 14%
Cystitis: 12%
Muscular pain or fibromyalgia: 4%
Irritable colon: 15%
Positivity to Ca 125: 100% average value 61,4U/ml (exclusion criterion if lower than 35 U/ml)
Dysmenorrhea VAS >= 5: 62%
Dyspareunia VAS>= 5: 30%
Chronic pelvic pain VAS>= at 5: 62%

### Serum examinations

| | |
|---|---|
| Dosage 17 Beta Estradiol on 21^{st} day of period average value group 2: | 176 pg/ml |
| Serum dosage PGE2 average value group 2: | 3305+/- 396ng/ml |

**3* month treatment VAS scale symptomatology values** with the composition of only two components, Linseed oil 1002mg (linolenic acid, 432 mg, linoleic acid, 172,8) and 5-Methyltetrahydrofolate calcium salt 400mcg,
Cephalea: 12%
Cystitis: 8%
Muscular pain (fibromyalgia): 4%
Irritable colon: 12%
Ca 125: 97% average value 52,6 U/ml
Dysmenorrhea VAS>= 5: 41%
Dyspareunia VAS>= 5: 37%
Chronic pelvic pain VAS >= 5: 45%

### Serum examinations

| | |
|---|---|
| Dosage 17 Beta Estradiol (on 21^{st} day of period) group 2: | 168pg/ml |
| Serum dosage PGE2 group 2: | 2605+/-396ng/ml |

### Placebo group like group I and II pre-treatment

Cephalea: 14%
Cystitis: 12%
Muscular pain or fibromyalgia: 4%
Irritable colon: 15%
Positivity to Ca 125: 100% average value 61,4 U/ml (exclusion criterion if lower than 35 U/ml))
Dysmenorrhea VAS >= 5: 62%
Dyspareunia VAS>= 5: 30%
Chronic pelvic pain VAS>= a 5: 62%

### Serum examinations

| | |
|---|---|
| Dosage 17 Beta Estradiol (on 21^{st} day of period) placebo group: | 164pg/ml |
| Serum dosage PGE2 average value placebo group: | 3481+/-462ng/l |

### Placebo group after 3-month treatment (placebo)

Cephalea: 13%
Cystitis: 12%
Muscular pain or fibromyalgia: 4%
Irritable colon: 15%
Positivity to Ca 125: 100% (exclusion criterion if lower than 35 U/ml)
Dysmenorrhea VAS >= 5: 60%
Dyspareunia VAS>= 5: 30%
Chronic pelvic pain VAS>= a 5: 60%

### Serum examinations

| | |
|---|---|
| Dosage 17 Beta Estradiol (on 21^{st} day of period) placebo group: | 154pg/ml |
| Serum dosage of PGE2 placebo group: | 3469+/-451ng/l |

## Claims

1. A composition comprising quercetin, an extract of a plant belonging to the genus curcuma or curcumin, an extract of a plant belonging to the species feverfew (*Tanacetum parthenium)* and a preparation comprising omega-3 and/or omega-6.

2. The composition according to claim 1 further comprising nicotinamide and/or 5-methyltetrahydrofolate calcium salt.

3. The composition according to claim 1 or 2 wherein said preparation comprising omega-3 and/or omega-6 is linseed oil.

4. The composition according to anyone of claims 1 to 3 wherein the amount of omega-3 is between 200 and 3000 mg and/or omega-6 is between 50 and 2000 mg and/or quercetin is between 50 and 1000 mg and/or feverfew is between 3 and 40 mg and/or curcuma is between 15 and 200 mg.

5. The composition according to anyone of claims 2 to 4 wherein the amount of nicotinamide is between 5 and 100 mg and/or 5-methyltetrahydrofolate calcium salt is between 50 and 800 mg.

6. Composition for oral use according to anyone of claims 1 to 5 in a form selected from capsule, soft capsule, tablet, pill, jelly, powder, granule, emulsion, solution, syrup.

7. The composition according to anyone of claims 1 to 6 wherein said composition is a food supplement, a nutraceutical, dietary and nutritional composition, a food product, a beverage, a neutraceutical, a medicament, a medicated food or a food for special medical purposes.

8. The composition according to anyone of claims 1 to 7 for use in the treatment of endometriosis.

9. The composition according to anyone of claims 1 to 8 for use in the treatment of one or more symptoms associated with endometriosis selected from acute and chronic pelvic pain, dyspareunia, dysmenorrhea, pain related to ovulation, pain related to urination, pain in the bowel functions, chronic physical fatigue, headache, female infertility and in reducing endometriosis outbreaks.

10. A kit comprising quercetin, extract of curcuma or curcumin, feverfew extract and a preparation comprising omega-3 and/or omega-6 ad optionally nicotinamide and/or 5-methyltetrahydrofolate calcium salt.

## Patentansprüche

1. Stoffgemisch umfassend Querzetin, ein Extrakt einer Pflanze, die zur Gattung Curcuma oder Curcumin gehört, ein Extrakt einer Pflanze, die zur Art Mutterkraut (Tanacetum phartenium) gehört, und eine Zubereitung umfassend Omega-3 und/oder Omega-6.

2. Stoffgemisch gemäß Anspruch 1 ferner umfassend Nikotinamid und/oder 5-Methyltetrahydrofolatkalziumsalz.

3. Stoffgemisch gemäß Anspruch 1 oder 2, wobei die Zubereitung, die Omega-3 und/oder Omega-6 umfasst Leinöl ist.

4. Stoffgemisch gemäß einem der Ansprüche 1 bis 3, wobei die Menge an Omega-3 zwischen 200 und 3000 mg ist und/oder an Omega-6 zwischen 50 und 2000 mg ist und/oder an Querzetin zwischen 50 und 1000 mg ist und/oder an Mutterkraut zwischen 3 und 40 mg ist und/oder an Curcuma zwischen 15 und 200 mg ist.

5. Stoffgemisch gemäß einem der Ansprüche 2 bis 4, wobei die Menge an Nikotinamid zwischen 5 und 100 mg ist und/oder an 5-Methyltetrahydrofolatkalziumsalz zwischen 50 und 800 mg ist.

6. Stoffgemisch zur oralen Verwendung nach einem der Ansprüche 1 bis 5 in einer Form ausgewählt aus Kapsel, Weichkapsel, Tablette, Pille, Gelee, Pulver, Granulat, Emulsion, Lösung oder Sirup.

7. Stoffgemisch gemäß einem der Ansprüche 1 bis 6, wobei das Stoffgemisch ein Nahrungsergänzungsmittel, ein Nutrazeutikum, ein Diät- und Nährstoffgemisch, ein Nahrungsmittelprodukt, ein Getränk, ein Neutrazeutikum, ein Medikament, ein medizinisches Nahrungsmittel oder ein Nahrungsmittel für besondere medizinische Zwecke ist.

8. Stoffgemisch gemäß einem der Ansprüche 1 bis 7 zur Verwendung bei der Behandlung von Endometriose.

9. Stoffgemisch gemäß einem der Ansprüche 1 bis 8 zur Verwendung bei der Behandlung von einem oder mehreren mit Endometriose assoziierten Symptomen, ausgewählt aus akuten und chronischen Beckenschmerzen, Dyspareunie, Dysmenorrhoe, Schmerzen im Zusammenhang mit dem Eisprung, Schmerzen im Zusammenhang mit dem Wasserlassen, Schmerzen der Darmfunktionen, chronischer körperlicher Müdigkeit, Kopfschmerzen, weiblicher Unfruchtbarkeit und beim Verringern von Endometrioseausbrüchen.

10. Set umfassend Querzentin, Curcuma- oder Curcuminextrakt, Mutterkrautextrakt und ein Präparat umfassend Omega-3 und/oder Omega-6 und optional Nikotinamid und/oder 5-Methyltetrahydrofolatkalziumsalz.

## Revendications

1. Composition comprenant de la quercétine, un extrait d'une plante appartenant au genre curcuma ou curcumine, un extrait d'une plante appartenant à l'espèce grande camomille (*Tanacetum parthenium*) et une préparation comprenant des oméga-3 et/ou oméga-6.

2. Composition selon la revendication 1, comprenant en outre du nicotinamide et/ou un sel calcique de 5-méthyltétrahydrofolate.

3. Composition selon la revendication 1 ou 2, dans laquelle ladite préparation comprenant des oméga-3 et/ou oméga-6 est l'huile de graines de lin.

4. Composition selon l'une quelconque des revendications 1 à 3, dans laquelle la quantité d'oméga-3 est comprise entre 200 et 3 000 mg et/ou d'oméga-6 est comprise entre 50 et 2 000 mg et/ou de quercétine est comprise entre 50 et 1 000 mg et/ou de camomille romaine est comprise entre 3 et 40 mg et/ou de curcuma est comprise entre 15 et 200 mg.

5. Composition selon l'une quelconque des revendications 2 à 4, dans laquelle la quantité de nicotinamide est comprise entre 5 et 100 mg et/ou de sel calcique de 5-méthyltétrahydrofolate est comprise entre 50 et 800 mg.

6. Composition pour une utilisation orale selon l'une quelconque des revendications 1 à 5 sous une forme choisie parmi la capsule, la gélule molle, le comprimé, la pilule, la gelée, la poudre, le granulé, l'émulsion, la solution, le sirop.

7. Composition pour une utilisation orale selon l'une quelconque des revendications 1 à 6, dans laquelle ladite composition est un complément alimentaire, un nutraceutique, une composition diététique et nutritionnelle, un produit alimentaire, une boisson, un nutraceutique, un médicament, un aliment médicalisé ou un aliment destiné à des fins médicales spécifiques.

8. Composition selon l'une quelconque des revendications 1 à 7 pour l'utilisation dans le traitement de l'endométriose.

9. Composition selon l'une quelconque des revendications 1 à 8 pour l'utilisation dans le traitement d'un ou de plusieurs symptômes associés à l'endométriose choisis parmi la douleur pelvienne aiguë et chronique, la dyspareunie, la dysménorrhée, la douleur liée à l'ovulation, la douleur liée à la miction, la douleur au niveau des fonctions intestinales, la fatigue physique chronique, les maux de tête, l'infertilité féminine et la réduction des crises d'endométriose.

10. Kit comprenant de la quercétine, un extrait de curcuma et de curcumine, un extrait de grande camomille et une préparation comprenant des oméga-3 et/ou oméga-6 en plus éventuellement du nicotinamide et/ou du sel calcique de 5-méthyltétrahydrofolate.
